# EUROPEAN PATENT SPECIFICATION

(11) **EP 2 255 204 B1**
(45) Date of publication and mention of the grant of the patent: **06.11.2013**
(21) Application number: 09722701.1
(22) Date of filing: 22.03.2009
(51) Int. Cl.: G01N 33/68, G01N 33/58

(54) **METHOD AND KIT FOR DIAGNOSIS OF MALE FERTILITY**
VERFAHREN UND KIT ZUR DIAGNOSE VON MÄNNLICHER FRUCHTBARKEIT
PROCÉDÉ ET NÉCESSAIRE POUR LE DIAGNOSTIC DE LA FERTILITÉ MASCULINE

(30) Priority: 20.03.2008 US 64685
(43) Date of publication of application: 01.12.2010
(73) Proprietor: Zanbar, Tidhar, 49935 Kfar Sirkin (IL)
(72) Inventor: Zanbar, Tidhar, 49935 Kfar Sirkin (IL)
(74) Representative: Fleuchaus, Andrea
(86) International application number: PCT/IL2009/000320
(87) International publication number: WO 2009/116051

(56) References cited:
- WO-A-00/09648
- WO-A-99/66331
- WO-A-2004/053465
- US-A- 5 434 057
- US-A- 5 935 800

## Description

### FIELD OF THE INVENTION

This invention relates to a method and kit for diagnosing male fertility.

### BACKGROUND OF THE INVENTION

Approximately 15% of couples attempting their first pregnancy meet with failure. Data available over the past twenty years reveal that in approximately 30% of these cases pathology is found in the man alone, and in another 20% both the man and woman are abnormal. Therefore, the male factor is at least partly responsible in about 50% of infertile couples.

Male infertility is a significant medical problem affecting a large proportion of the population. About half of the causes of male infertility exhibit usually no specific cause that can be identified, even after thorough evaluation.

Three of the main factors associated with the fertility of the male are typically recognized as being detrimental for successful insemination: the number or percentage of motile sperm, the speed of movement and the direction thereof. The most encountered defects are related to the motion characteristics of the sperm and specifically to the ability of the sperm to move forward, a movement that is governed and caused by effective beating of their tail (*flagellum*). This movement or so-called progressive motility of the sperm is considered by most IVF laboratories as being the most predictive indicator of male infertility.

According to the World Health Organization (WHO) criteria for male fertility, the primary factors governing male fertility are semen volume, sperm cell density, motility, forward progression, and morphology. The WHO guidance for 'normality' is 10 million motile sperm per milliliter.

Traditionally, progressive motility (forward progression) has been determined and quantified by (light) microscopic analysis of the sample by a laboratory trained technician. The sample is observed and several aspects of the sperm cell composition are quantified, such as percent motility, density, percent morphologically abnormal, and forward progression. A number of methods exist for assessing the motility and number of sperm in a sample. As mentioned, common in the art are microscopic analysis methods, which are typically carried out in a hospital or commercial laboratory. More recently; a number of test kits which are intended to simplify the detection of sperm have been developed.

W095/29188 discloses a test based on antibodies to the SP-10 antigen of human sperm. EP-A-0387873 discloses a kit which uses solid beads to which are bound antibodies specific to the human spermatozoon acrosome. The beads are mixed with a sample, incubated, separated, washed, and the number of sperm bound to the beads is measured, preferably by examination with the aid of a microscope. W0 97/40386 discloses a kit which is based on immunodetection of the-+kL, a human epididymal sperm protein. Sperm in a sample are washed three times by centrifugation in Dulbecco-phosphate buffered saline. The samples are then heat denatured at 95°C, centrifuged at 14000g, and the supernatants are then used for analysis.

Of late, single use, over-the-counter products that can achieve basic semen analysis are becoming available. Colorimetric assays for testing for semen quality are known in the art. For example, Genosis Ltd. (Kingston upon Thames, England) has produced a product sold under the trademark "Fertell" disclosed in International Publication No. WO/2001/060968. The Fertell Male fertility test challenges the sperm to swim up a fluid-filled chamber, mimicking the process that the sperm would go as it tries to reach the egg. The presence of sperm is detected with anti-CD59 monoclonal antibody conjugated with colloidal gold. Sperm bound to the antibody is trapped on a nitrocellulose strip while unreacted conjugate is washed from the strip with excess buffer

International Publication No. WO 99/66331 discloses a method and kit for separating motile sperm from non-motile sperm and/or sperm with reduced motility in a sample or for detecting motile sperm. The method of the invention comprises depositing a sample of sperm onto a porous layer of thickness 25-2,500 microns and allowing any motile sperm contained in the sample to migrate through the layer to a detection zone, where the motile sperm is detected.

US Publication No. 2005287571 discloses methods for evaluating male fertility, based on the level of EP2D bound to sperm, and also kits for evaluating male fertility.

### SUMMARY OF THE INVENTION

One of the major disadvantages of the diagnostic kits and methods disclosed in the art is that they do not distinguish between motile and non-motile sperm in a simple, rapid and accurate way. Moreover, many of the known diagnostic kits involve components which increase the cost of the kit available to the consumer and/or procedures which do not lend themselves to home use.

The inventor of the present invention has now successfully developed a method and kit for detecting the presence of sperm having the required progressive motility and the quantification thereof. The method and kit of the present invention have the following benefits in comparison to existing or known methods and kits for the diagnosis of male fertility:
**(1)** the diagnostic method may be carried out outside of a medical facility and in the comfort of the male subject's private home, thus overcoming the initial embarrassment of going to such a facility and having to produce a sample for analysis there;
**(2)** the diagnostic method does not require the involvement of a medical personnel in its execution or analysis (i.e., the presence or absence of motile sperm in a sample);
**(3)** the execution of the diagnosis and the analysis of the results may take only a few minutes;
**(4)** a large volume of sperm is not needed for the successful execution of the method;
**(5)** the diagnostic method may be carried out at any time of the subject's choosing;
**(6)** the diagnosis may be carried out at an early stage in a couple's relationship allowing them to identify the existence of fertility problems;
**(7)** the kit used for carrying out the method of the invention is inexpensive;
**(8)** the kit may be used both for determining of male fertility and also for determining the ratio of sperm having progressive motility to non-motile sperm;
**(9)** the kit may be used for determining the pH of sperm, other cells present in semen (such as leucocytes and immature sperm cells) and seminal fluid providing data of the semen, its viability and fertility;
**(10)** the kit may be used for determining the concentration of cells in the semen;
**(11)** the kit may optionally also include means for sperm washing prior to preparing the sperm for use in intra uterus sperm injection (IUI), allowing the sperm a better chance for survival and fertilization; and
**(12)** the kit and method of the invention may be used not only to analyze human sperm but also animal sperm in the field away from a laboratory set up.

Thus, in a first aspect of the present invention there is provided a method for diagnosing male fertility, said method comprising:
**(a)** placing a sample of sperm (e.g., obtained from the subject immediately prior to diagnosis or at any time period prior to diagnosis) in the vicinity (e.g., not in direct contact) of an agent capable of changing at least one of its measurable properties when in contact with a sample containing sperm;
**(b)** allowing sufficient time for the sperm in said sample to migrate towards said agent and come into contact therewith; and
**(c)** determining the presence or absence of sperm having progressive motility in said sample,
wherein a change in at least one of said measurable properties of said agent indicates the presence of sperm having progressive motility and wherein said agent is an indicator selected from Bradford, Biuret reagent and an indicator obtained by a Lowry protein assay.

The method also comprises, in some embodiments, determining the number of sperm having progressive motility thereby diagnosing the male's fertility.

In some embodiments of the invention, the sample of sperm is placed onto a physical barrier, which separates between the sample and the agent and which allows the migration of the sperm therethrough. The sample is typically the ejaculate, namely semen containing sperm retrieved by the male subject or through medical manipulation or where the fertility of a non-human male is to be evaluated the sperm sample may be obtained by methods known to those skilled in the art.

The sample may also be any sample containing the subject's semen or sperm. This sample may be placed (deposited) directly on the barrier by the male subject through active ejaculation or indirectly after it has been collected by the subject or a medical personal.

A sperm sample may be a sample obtained directly from a male subject, from a female vagina or an artificial vagina said sample being of a volume sufficient for effective analysis. In some embodiments, the volume is smaller than 0.3 ml, and in other embodiments, the volume is larger than 0.3 ml.

In further embodiments, the sperm sample used in the kit and methods of the invention is a fresh sperm sample. In some other embodiments, the sperm sample is a stored semen sample. Any storing methods may be used for storing the semen sample, e.g. freezing the semen sample, prior to diagnosis. A thawed semen sample may be filtered for removing substrate used when freezing the sperm using any method for filtering sperm known in the art.

The physical barrier used in the kit of the invention is selected to have at least one of the following features:
**(i)** a viscosity sufficient to provide a reliable intact barrier;
**(ii)** fluidity sufficient to facilitate sperm motility therethrough;
**(iii)** ability to prevent sperm sticking to sperm or to any component of the kit; and
**(iv)** having no deleterious effect on the sperm.

The physical barrier which is in contact with the sample, on one side thereof, and the reagent, on another side thereof, allows the placement of the sample in close proximity or vicinity to the reagent which is used for the qualitative and/or quantitative analysis. In some embodiments, where the kit includes at least two barriers, at least one of the barriers is in contact with the sample and/or at least one other is in contact with the reagent. One or more additional barriers may also be present. Any of the barrier materials should be of sufficient volume or thickness so as not to allow migration or diffusion of any component of the sample therethrough other than the sperm. In fact, the ability of the motile sperm to cross the barrier has to do with its ability to actively move forward and not on its ability to passively diffuse into or through the barrier material.

As the inventor has demonstrated, seminal fluid which does not contain progressively motile sperm does not affect any change in at least one of the measurable properties of the reagent as none of the components of the fluid are capable of crossing the barrier.

Additionally, the physical barrier is one which does not interact with the sperm in the sample, nor with any component of the sample containing the sperm and which does not induce any change in at least one of the measurable properties of said agent.

In some embodiments, the barrier is a liquid barrier. When a liquid barrier is used said barrier may include suspensions comprising of various amounts of polyvinylpyrrolidone (PVP), which may be mixed in oil, water or a mixture thereof. Typically, the concentration of PVP is between 0.05 and 0.5 mg/ml and in some embodiments, between 0.1 to 0.3 mg/ml. The suspension may also comprise dispersing agents or other components such as sodium carboxymethyl cellulose, hyaluronate, dioctyl calcium sulfosuccinate or other materials which can be combined in said suspension and enhance the desired properties of the barrier used in the kit of the invention as described herein.

The liquid barrier may also comprise oil such as a mineral oil, glycerol, propylene glycol or mucus. In some embodiments, the barrier comprises or is a mineral oil, or such as that suitable for use with tissue cultures.

In other embodiments, the barrier is a solid, semi solid, or a gellous barrier.

The barrier may be a single-component barrier or a multi-component barrier, constructed of at least two different barrier materials, e.g., two different oils, each being immiscible in the other, two different solids (mixed or layered), an oil barrier and a solid barrier, etc. The various barrier materials may be constructed in relation to each other, for example, according to their viscosity, porosity, and so on.

In some embodiments, the barrier is constructed of two different materials: the first being a mineral oil layer and the second being a solid matrix such as a solid net having pores of a size selected to allow migration of sperm there through. The solid net may be placed between the barrier layer and the agent or above the barrier layer, thereby separating between the barrier material and the sample containing the sperm.

Typically, when the solid matrix is used, it should have pores ranging in size between 50 and 200 microns.

In some embodiments, the solid net is a silk-based net. One such commercially available net is Nitrex.

In other embodiments, a liquid PVP barrier (comprising varying ratios of water/oil/PVP) may be combined with a mineral oil layer or with a solid matrix.

The method or kit of the invention, as further discussed below, does not require the use or presence of any one additional chemical or physical means to prevent the migration of non-motile or sperm with reduced motility through the barrier in the direction of the agent. It turns out, as will be demonstrated below, that the barrier material and/or the construction employed is sufficient to allow migration of substantially only sperm having progressive motility.

Within the scope of the present invention the "***agent***" is a chemical which changes any one measurable, e.g., physical property in contact with the sperm. Such physical property may be selected amongst the following measurable properties: color, consistency, physical state (e.g., solid to liquid), any one electrical property or any one measurable spectroscopic property. In some embodiments, the change in the physical property is noticeable by the naked eye. In some other embodiments, the change in the physical property is identified and/or measured spectroscopially.

Typically, the agent will not change any one of said at least one measurable properties in contact with other components (namely reagents) employed in the method or kit, e.g., the barrier material.

In some embodiments, the physical property is color and the change upon contact with the sperm is identified and/or measured by the naked eye.

In other embodiments, the agent is an indicator which changes its color and/or shade (hue) in contact with the sperm, said change in color and/or shade is measurable qualitatively and/or quantitatively, allowing both or either of diagnosing the male infertility and determining the percentage of sperm exhibiting progressive motility. The agent may be for example one or more reagents commercially available under the names Bradford, Biuret, and Lowry. These assay reagents may be used as commercially available or may be prepared according to procedures known in the art.

In some embodiments, the indicator is Bradford.

The "***Bradford***" is a spectroscopic analytical assay used to measure the concentration of protein in a sample depending on the amino acid composition of the measured protein. The assay is based on an absorbance shift in the dye Coomassie. The Bradford reagent product consists of Brilliant Blue G in phosphoric acid and methanol. Thus far, Bradford assay has not been reported for the detection and/or quantification of sperm.

The change in the color of the Bradford on contact with sperm is typically from grey-violate (or from any initial color) to any shade of blue (or any other color shade depending on the initial color) depending on the presence or absence of sperm in the sample and on the amount of motile sperm (typically measurable in percent motility). As will be shown below, the changes in color may be an indication of the number of motile sperm, independently of the amount of sample which is used in the method.

The intensity of the color change may be compared to a pre-calibrated color reference which may be part of the diagnostic kit. Thus, the method of the invention may also comprise the step of comparing the color of the agent in contact with the sperm to a pre-calibrated color reference for the qualitative and/or quantitative determination. For example, by comparing the resulting color to the pre-calibrated reference, the male subject will be able to identify whether the sperm concentration in the test sample is above the normal count of sperm in a specified volume, for example, above 20 million/mL (which may be regarded as a positive test) or below 20 million/mL (which may be regarded as a negative test).

In other embodiments, the indicator is a Biuret reagent. The "***Biuret reagent***" is made of potassium hydroxide (KOH) and copper (II) sulfate (CuSO₄), together with potassium sodium tartrate KNaC₄H₄O₆·4H₂O. The blue reagent turns violet/pink in the presence of the sperm.

In yet further embodiments, the indicator is the reagent employed in the Lowry protein assay.

In some further embodiments, the concentration of the sperm in the sample is determined by using a first antibody specific for a sperm tissue-specific protein antigen, under conditions which permit said antibody to bind any antigen present in said sample, and determing the frequency of said binding, with or without a secondary antibody, wherein the frequency of occurrence of said binding is indicative of the total sperm concentration in said sample. The antibodies used herein may be any type of sperm detecting antibody know in the art such as sperm centriole antibodies, antibodies against seminal fluid heparin-binding proteins, anti-Dyenin (cytoplasmic or axonemal) antibidies and antibodies that bind to the acrosome region of the sperm.

In some cases when antibody detection is used, the sperm sample is frozen immediately upon collection to prevent degradation of the antigens present in said sample.

The active movement of the sperm through the barrier in the direction of the agent may be achieved by gravity, for example, by placing the sample at a position above the agent, thereby allowing movement of the sperm in the direction of gravity. In other embodiments, the migration is forced migration by which the sperm are forced to cross the barrier. Such forced migration allows the construction of horizontal kits wherein the sample is placed substantially to the side of the indicator agent, e.g., to the right or left of the indicator agent and not necessarily above the agent as in the vertical construction.

In some embodiments, the agent is positioned in the vicinity of at least one chemoattractant that will aid the sperm in their movement towards the agent.

In another one of its aspects, the method of the invention further allows for combining the diagnosis of fertility as herein disclosed with a sperm washing step whereby prior to the fertility diagnosis, or in parallel thereto an amount of sperm is washed, in accordance with any one procedure known in the art (see for example http://sharedjourney.com/iui/sperm_washing.html) to prepare sperm for IUI, thereby allowing for a better chance for sperm survival and fertilization. Such a method has the advantage that a single original amount of ejaculate is divided into two samples, one being used to assay fertility potential and the other prepared for IUI.

As known in the art, sperm washing separates sperm cells from semen, helping to rid the sample of dead or slow-moving sperm as well as additional chemicals that may impair fertilization. Thus, once sperm has been washed, it can be used during IUI for achieving pregnancy.

The sperm washing procedure may include the use of a sperm wash solution for removal of chemicals, which may cause adverse reactions in the uterus. The sperm wash solution employed may be any sperm wash solution known in the art. For example, the solution may comprise density gradient compounds, such as dextran, iodixanol, sucrose, polymers, nycodenz, or polyvinylpyrrolidone, coated silica a balanced salt solution and a macromolecule.

In yet another aspect there is described a method for determining pregnancy potential of a sample or identifying amongst two or more sperm samples those having pregnancy potential, said method comprising:
**(i)** obtaining at least one sperm sample from a male subject (e.g., a donor), wherein additional samples (e.g., from the same subject) may be obtained over time;
**(ii)** obtaining an aliquot from each of said at least one sperm samples;
**(iii)** testing said aliquot according to the method of the invention to determine pregnancy potential; and
(iv) determining whether the sample has pregnancy potential or the at least one sperm sample having pregnancy potential.

In some embodiments, said method further comprises a sperm washing step as described herein. In some further embodiments, the sperm sample having pregnancy potential, namely that which has the necessary count of motile sperm, may be used for insemination, e.g., utilizing an artificial reproductive technology (ART).

The diagnostic method is used for screening sperm samples for detecting the pregnancy potential of sperm samples. The screening is, in some embodiments, based on determining motile sperm count. This method may be utilize to identify a sample having pregnancy potential amongst a great number of such samples or to determine changes (e.g., fluctuations) in sperm quality (e.g., percent motile sperm, etc) of an individual over time.

Thus, the present invention also relates to a method for identifying potentially infertile males or evaluating male fertility following an event which may influence the subject's fertility, e.g., radiation therapy, vasectomy, injury to reproduction organ, age, general medical condition, and other events identified by a medical practitioner. The method of the invention, particularly the quantitative aspect thereof may be employed to estimate the number of motile sperm in the sample. For example, quantization of less than about 20 million sperm/mL may be taken to indicate that the sample has low pregnancy potential; greater than about 20 million sperm/mL and less than about 40 million sperm/mL may indicate that the sample has borderline pregnancy potential; and greater than about 40 million sperm/mL may indicate that the sample has high pregnancy potential.

In still yet another aspect, the invention provides sperm diagnostic kits which comprise a number of simple reagents and devices, in some embodiments, packaged in a box. The kit for use in a method for determining male fertility or any other method disclosed herein, comprises:
**(i)** at least one container for holding a sample containing sperm;
**(ii)** a receptacle having at a first end thereof a first compartment containing at least one agent capable of changing at least one of its measurable properties when in contact with a sample containing sperm, a second compartment at a second end of said receptacle for holding the sample, said first compartment being separated from said second compartment by a barrier in such a way to allow crossing of the sperm in the sample but not direct contact between said sample and said agent; and wherein said agent is an indicator selected from Bradford, Biuret reagent and an indicator obtained by a Lowry protein assay; and
**(iii)** instructions of use.

The term "***compartment***" as used herein may be any chamber separated from another by physical means or any volume of the receptacle which is not separated from another volume by any one physical means.

The kit may also comprise at least one measuring unit for holding a sperm sample and separating it into two or more fractions. The kit may further comprise at least one additional receptacle holding a sperm washing solution to permit sperm washing.

The kit may further include a sperm isolation means to facilitate collection (e.g., funnel shaped).

The kit may further comprise at least one pre-calibrated color reference for determining the concentration of the sperm in the sample, as detailed above.

The kit of the invention may be operated by the male undergoing the diagnosis, devoiding the involvement of a medical practitioner.

The method and kits of the invention can also be useful for determining the fertility of animals, particularly for the animals with the sperm concentration and sizes of the same order of magnitude as for those of men, such as dogs, bull, stallion, rabbit etc.

### BRIEF DESCRIPTION OF THE DRAWINGS

In order to understand the invention and to see how it may be carried out in practice, embodiments will now be described, by way of non-limiting example only, with reference to the accompanying drawings, in which:
**Fig. 1** is a schematic representation of a general kit construction according to one embodiment of the present invention.
**Figs. 2A** and **2B** are schematic representations of a two embodiments of a kit of the present invention.
**Fig. 3** is a schematic illustration of a further embodiment of a kit of the present invention.
**Fig. 4** is a schematic illustration of a yet another embodiment of a kit of the present invention.

### DETAILED DESCRIPTION OF EXEMPLARY EMBODIMENTS

The herein disclosed methods and kits are easy to perform and operate and may be completed in less than about 15 minutes. As stated herein, the methods and kits provide information which may be useful, for example, in monitoring the effect of changes in such factors as diet, sleep, exercise, exposure to smoke or other carcinogens, and intake of alcohol or drugs on the fertility of sperm samples produced thereafter.

Also, the methods and kits can indicate whether a particular male fertility or contraceptive treatment has been effective or whether sperm subsequently obtained from the same male has the ability to initiate pregnancy. In addition, the methods and kits may be useful for determining whether a particular sperm sample is suitable for use in an ART.

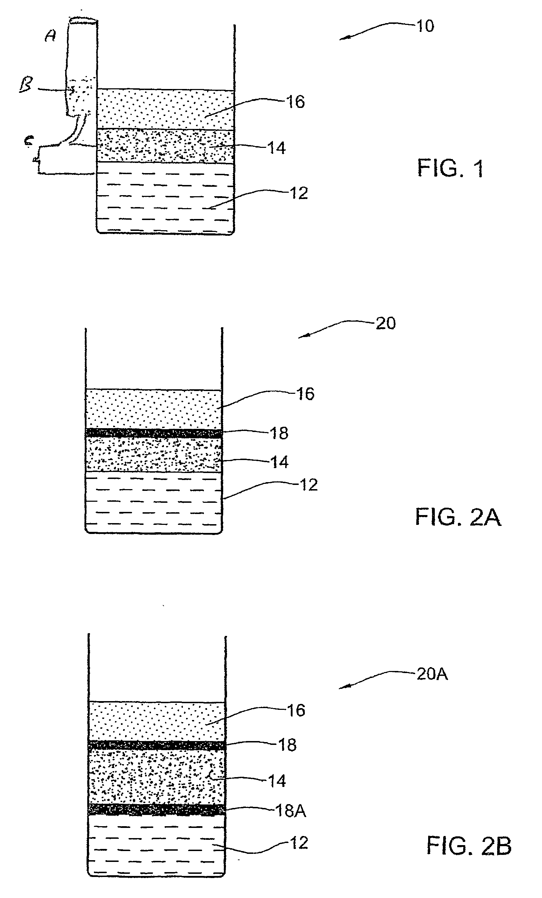

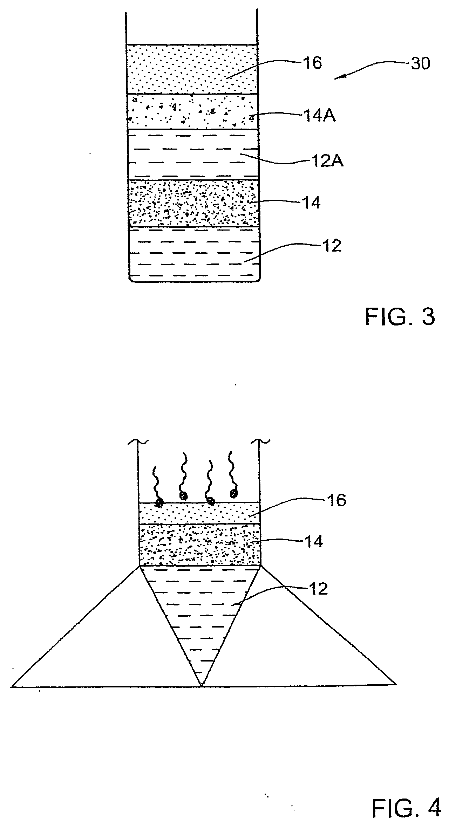

One exemplary embodiment of a kit of the present invention is illustrated in **Fig. 1**. A container **10** for holding the agent, e.g., an indicator **12**, the barrier **14** on top of which a sample **16** of ejaculate from a male subject (which may contain both motile and non-motile sperm) is placed. The container **10** may have a base of different shapes, as exemplified in the structure of **Fig. 4**. Although, the container **10**, illustrated as having a substantially rectangular shape, can be configured to have any selected shape, such as a funnel-like configuration to assist the male donor in collecting and capturing a sperm sample. The container **10** can be made from any suitable biocompatible material, such as glass or plastic. The container may be of any size. Upon the addition of the sperm sample **16** into the contain **10**, on top of the barrier layer **14**, the motile sperm begins migrating through the barrier **14** in the direction of the indicator agent **12** contained in the bottom of the container **10**. After a sufficient period of time has elapsed (ca. **15** minutes) and provided that the sample contains motile sperm, a color change should be observed in the indicator agent **12**. Without being separated by any physical dividers, each of the volumes **12, 14** and **16** is referred herein as a compartment.

The container may optionally comprise an additional receptacle **A** to hold a sperm wash solution **B** for ridding the sample of dead or slow-moving sperm as well as additional chemicals that may impair fertilization prior to, e.g., IUI procedure. Thus, a sperm sample may be divided and one fraction may be utilized for fertility diagnosis while a second fraction may be washed employing a sperm wash solution as described herein. Following a sperm wash step the second sperm sample fraction may be transferred to an additional receptacle **C** where it is stored for use in IUI. In some embodiments of the invention, the receptacle **A** has a threaded outer surface on top for engaging a syringe or a disposable pipette used to place the sperm sample.

In practicing the invention, larger or smaller receptacles may be utilized and still be within the scope of the invention. These other sizes allow for different amounts of sample, sperm wash solution and barrier material, in order to maximize sperm diagnosis/washing for the particular diagnostic purpose and the IUI procedure used.

Alternatively, as shown in **Fig. 2A**, the sperm sample **16** is added into a container **20** having an indicator **12** contained in the bottom of the container **10**, onto which a barrier layer **14** is placed, bordered by, e.g., a silk layer **18** which protects the barrier material from the added sperm sample. A second silk layer **18A** may also be placed between the indicator agent **12** and the barrier layer **14** as shown in **Fig. 2B**.

In another exemplary embodiment of the kit of the invention, illustrated in **Fig. 3**, a container **30** is provided for holding an indicator agent **12** at the bottom of the container, a barrier layer **14** placed between the indicator agent **12** and a second volume of another indicator agent **12A** which may or may not be identical to the indicator **12**, a second barrier layer separating between the indicator **12A** and the sample of sperm **16**. As before, each of the barrier layers **14** and **14A** may be bordered on one or both of its sides with a solid matrix such as a silk layer.

As indicated above, the container may be substantially vertical or horizontal with the different compartments constructed one to the side of the other is a sequence similar to any one of the embodiments above.

## Claims

1. A method for diagnosing male fertility, said method comprising
(a) placing a sample of sperm in the vicinity of an agent capable of changing at least one of its measurable physical properties when in contact with a sample containing sperm;
(b) allowing sufficient time for the sperm in said sample to migrate towards said agent and come in contact therewith; and
(c) determining the presence or absence of sperm having progressive motility in said sample,
wherein a change in at least one of the measurable physical properties of said agent indicates the presence of sperm having progressive motility, and wherein said agent is an indicator selected from Bradford, Biuret reagent and an indicator employed by a Lowry protein assay.

2. The method according to claim 1, wherein said sample of sperm is placed onto a physical barrier, which separates between the sample and the agent and which allows the migration of the sperm there through.

3. The method according to claim 2, wherein said barrier is a liquid barrier, a solid barrier, a semi solid barrier or gellous.

4. The method according to claim 3, wherein said solid barrier is a solid matrix having pores ranging in size between 50 and 200 microns.

5. The method according to claim 4, wherein said matrix is a silk based net.

6. The method according to any one of claims 2 to 5, wherein said barrier is a single component barrier or a multi-component barrier.

7. The method according to any one of claims 2 to 6, wherein said barrier is in the form of a suspension comprising at least one polyvinylpyrrolidone, oil, water and a mixture thereof.

8. The method according to claim 7, wherein said oil is mineral oil.

9. The method according to any one of claims 2 to 8, wherein said barrier further comprising at least one of glycerol, propylene glycol and mucus.

10. The method according to any one of the preceding claims, wherein said change in at least one of the physical properties of said agent is identifiable and/or measurable by the naked eye.

11. The method according to any one of the preceding claims, said method further comprising a step of determining the concentration of sperm in said sample.

12. The method according to any one of the preceding claims, wherein said agent is positioned in the vicinity of at least one chemo-attractant.

13. A kit for use in a method for determining male fertility, said kit comprising:
(a) at least one container for holding a sample containing sperm;
(b) a receptacle having at a first end thereof a first compartment containing at least one agent capable of changing at least one of its measurable physical properties when in contact with a sample containing sperm, a second compartment at a second end of said receptacle for holding the sample, said first compartment being separated from said second compartment by a barrier in such a way to allow liquid communication but not direct contact between said sample and said agent;
(c) instructions of use,
and wherein said agent is an indicator selected from Bradford, Biuret reagent and an indicator employed by a Lowry protein assay.

## Patentansprüche

1. Verfahren zum Diagnostizieren der männlichen Fertilität, wobei das Verfahren umfasst:
(a) Platzieren einer Spermaprobe in der Nähe eines Wirkstoffes, der in der Lage ist, bei Kontakt mit einer Sperma enthaltenden Probe wenigstens eine seiner messbaren physikalischen Eigenschaften zu verändern;
(b) Verstreichenlassen einer ausreichenden Zeit, während derer das Sperma in der Probe zu dem Wirkstoff migrieren kann und mit demselben in Kontakt gelangen kann; und
(c) Bestimmen des Vorhandenseins oder Nichtvorhandenseins von Sperma mit progressiver Motilität in der Probe,
wobei eine Veränderung wenigstens einer der messbaren physikalischen Eigenschaften des Wirkstoffs das Vorhandensein von Sperma mit progressiver Motilität anzeigt und wobei der Wirkstoff ein Indikator ist, der aus Bradford, Biuret-Reagens und einem durch einen Lowry-Protein-Assay verwendeten Indikator ausgewählt ist.

2. Verfahren gemäß Anspruch 1, wobei die Spermaprobe auf eine physische Barriere platziert wird, welche eine Trennung zwischen der Probe und dem Wirkstoff schafft und welche die Hindurchmigration des Spermas zulässt.

3. Verfahren gemäß Anspruch 2, wobei die Barriere eine flüssige Barriere, eine feste Barriere, eine halbfeste Barriere oder gelartig ist.

4. Verfahren gemäß Anspruch 3, wobei die feste Barriere eine Feststoffmatrix mit Poren ist, deren Größe im Bereich zwischen 50 und 200 Mikrometern liegt.

5. Verfahren gemäß Anspruch 4, wobei die Matrix ein Netz auf Seidenbasis ist.

6. Verfahren gemäß einem der Ansprüche 2 bis 5, wobei die Barriere eine Einkomponentenbarriere oder eine Mehrkomponentenbarriere ist.

7. Verfahren gemäß einem der Ansprüche 2 bis 6, wobei die Barriere die Form einer Suspension hat, die wenigstens ein Polyvinylpyrrolidon, Öl, Wasser und eine Mischung daraus umfasst.

8. Verfahren gemäß Anspruch 7, wobei das Öl mineralisches Öl ist.

9. Verfahren gemäß einem der Ansprüche 2 bis 8, wobei die Barriere weiterhin wenigstens eines von Glycerol, Propylenglycol und Mucus umfasst.

10. Verfahren gemäß einem der vorhergehenden Ansprüche, wobei die Veränderung wenigstens einer der physikalischen Eigenschaften des Wirkstoffs mit bloßem Auge erkennbar und/oder messbar ist.

11. Verfahren gemäß einem der vorhergehenden Ansprüche, wobei das Verfahren weiterhin einen Schritt einer Bestimmung der Konzentration von Sperma in der Probe umfasst.

12. Verfahren gemäß einem der vorhergehenden Ansprüche, wobei der Wirkstoff in der Nähe wenigstens eines Chemoattraktans angeordnet ist.

13. Kit zur Verwendung in einem Verfahren zum Bestimmen der männlichen Fertilität, wobei das Kit umfasst:
(a) wenigstens einen Behälter zum Aufnehmen einer Sperma enthaltenden Probe;
(b) ein Gefäß mit, an einem ersten Ende desselben, einem ersten Abteil, das wenigstens einen Wirkstoff enthält, welcher in der Lage ist, bei Kontakt mit einer Sperma enthaltenden Probe wenigstens eine seiner messbaren physikalischen Eigenschaften zu verändern, und einem zweiten Abteil an einem zweiten Ende des Gefäßes zum Aufnehmen der Probe, wobei das erste Abteil von dem zweiten Abteil durch eine Barriere auf solche Weise getrennt ist, dass eine Flüssigkeitskommunikation, jedoch kein direkter Kontakt zwischen der Probe und dem Wirkstoff zugelassen ist;
(c) eine Gebrauchsanweisung,
und wobei der Wirkstoff ein Indikator ist, der aus Bradford, Biuret-Reagens und einem durch einen Lowry-Protein-Assay verwendeten Indikator ausgewählt ist.

## Revendications

1. Méthode de diagnostic de la fertilité masculine, ladite méthode comprenant :
(a) le placement d'un échantillon de sperme à proximité d'un agent apte à changer au moins une de ses propriétés physiques mesurables lorsqu'il est en contact avec un échantillon contenant du sperme ;
(b) l'autorisation de temps suffisant pour que le sperme dans ledit échantillon puisse migrer vers ledit agent et entrer en contact avec celui-ci ; et
(c) la détermination de la présence ou de l'absence de sperme ayant une motilité progressive dans ledit échantillon,
dans laquelle un changement dans au moins une des propriétés physiques mesurables dudit agent indique la présence de sperme possédant une motilité progressive et dans laquelle ledit agent est un indicateur sélectionné à partir du réactif de Bradford, Biuret et un indicateur employé par un essai protéinique de Lowry.

2. Méthode selon la revendication 1, dans laquelle ledit échantillon de sperme est placé sur une barrière physique qui fait séparation entre l'échantillon et l'agent et qui autorise la migration du sperme vers celui-là.

3. Méthode selon la revendication 2, dans laquelle ladite barrière est une barrière liquide, une barrière solide, une barrière semi-solide ou un gel.

4. Méthode selon la revendication 3, dans laquelle ladite barrière solide est une grille solide possédant des pores rangés par taille entre 50 et 200 microns.

5. Méthode selon la revendication 4, dans laquelle ladite grille est un un filet à base de soie.

6. Méthode selon une quelconque des revendications 2 à 5, dans laquelle ladite barrière est une barrière à composant unique ou une barrière à plusieurs composants.

7. Méthode selon une quelconque des revendications 2 à 6, dans laquelle ladite barrière est sous la forme d'une suspension comprenant au moins un polyvinylpyrrolidone, de l'huile, de l'eau et un mélange de ceux-ci.

8. Méthode selon la revendication 7, dans laquelle ladite huile est de l'huile minérale.

9. Méthode selon une quelconque des revendications 2 à 8, dans laquelle ladite barrière comprend au moins soit de la glycérine, soit du glycol de propylène, soit du mucus.

10. Méthode selon une quelconque des revendications précédentes, dans laquelle ledit changement dans au moins une des propriétés physiques dudit agent est identifiable et/ou mesurable à l'oeil nu.

11. Méthode selon une quelconque des revendications précédentes, ladite méthode comprenant par ailleurs une étape de détermination de la concentration de sperme dans ledit échantillon.

12. Méthode selon une quelconque des revendications précédentes, dans laquelle ledit agent est positionné à proximité d'au moins un attracteur chimique.

13. Kit d'utilisation d'une méthode de détermination de la fertifilité masculine, ledit kit comprenant :
(a) au moins un flacon destiné à recevoir un échantillon contenant du sperme ;
(b) un réceptacle possédant une première extrémité dont un premier compartiment contient au moins un agent capable de changer au moins une de ses propriétés physiques mesurables lorsqu'il est en contact avec un échantillon contenant du sperme, un second compartiment à une seconde extrémité dudit réceptacle pour recevoir échantillon, ledit premier compartiment étant séparé dudit second compartiment par une barrière de manière à permettre une communication liquide mais pas un contact direct entre ledit échantillon et ledit agent ;
(c) des instructions d'utilisation,
et dans lequel ledit agent est un indicateur sélectionné à partir du réactif de Bradford, Biuret et un indicateur employé par un essai protéinique de Lowry.
